# EUROPEAN PATENT APPLICATION

(11) **EP 4 015 080 A1**
(43) Date of publication of application: **22.06.2022**
(21) Application number: 21205689.9
(22) Date of filing: 29.10.2021
(51) Int. Cl.: B01L 3/00, B01L 1/00, B01L 7/00

(54) **ALL-IN-ONE KIT FOR ON-SITE MOLECULAR DIAGNOSIS AND MOLECULAR DIAGNOSIS METHOD USING SAME**

(30) Priority: 17.12.2020 KR 20200177139; 08.10.2021 KR 20210134058
(71) Applicant: PHILMEDI Co., Ltd., Gyeonggi-do, Seongnam-si 13120 (KR)
(72) Inventor: LEE, Byung Hwan, 17086 Yongin-si (KR); KIM, Sejin, 13279 Seongnam-si (KR); SONG, Junkyu, 10362 Goyang-si (KR)
(74) Representative: Stolmár & Partner Patentanwälte PartG mbB

(57) **Abstract**

Proposed are an all-in-one kit for on-site molecular diagnosis and a molecular diagnosis method using the same. The all-in-one kit for on-site molecular diagnosis includes a first component including a sample pre-processing part, a nucleic acid adsorption part, and a nucleic acid amplification part, and a second component including a detection part. The sample pre-processing part includes a transfer member configured to transfer a lysis buffer where a nucleic acid is dissolved and is configured to pre-process a sample. The nucleic acid adsorption part is positioned on the sample pre-processing part and is configured to be slidable onto the nucleic acid amplification part. The nucleic acid amplification part is connected to the sample pre-processing part and is configured to elute the nucleic acid from the nucleic acid adsorption part and to amplify the eluted nucleic acid. The detection part is configured to transfer the nucleic acid amplified by the nucleic acid amplification part and to detect the nucleic acid. The all-in-one kit for on-site molecular diagnosis and the molecular diagnosis method using the same according to the present disclosure are advantageous in that the kit enables an ordinary person to perform molecular diagnosis without requiring the need for specialized equipment such as a centrifuge or pipette, ensures low contamination operation, and is cost effective.

## Description

### CROSS REFERENCE TO RELATED APPLICATION

The present application claims priority to Korean Patent Application Nos. 10-2020-0177139, filed December 17, 2020 and 10-2021-0134058, filed October 8, 2021, and the entire contents of which is incorporated herein for all purposes by this reference.

### BACKGROUND OF THE INVENTION

### Field of the Invention

The present disclosure relates to a kit for molecular diagnosis and a molecular diagnosis method using the same and, more particularly, to an all-in-one kit for on-site molecular diagnosis and a molecular diagnosis method using the same.

### Description of the Related Art

In the United States each year, over 1 billion infections occur, and many cases are mistreated as a result of inaccurate or delayed diagnosis. Many point-of-care (POC) tests suffer from poor sensitivity (30% to 70%), and much more sensitive tests, such as specific detection of nucleic acids or molecular tests associated with pathogenic targets, are only available in the laboratory.

However, devices and techniques for performing laboratorybased molecular diagnosis tests require skilled personnel, regulated infrastructure, and expensive instruments. Because laboratory equipment typically processes many samples at once, central laboratory tests are often performed in batches. A method for the processing of test samples typically involves processing all samples collected over a period of time (e.g., one day) in one large-scale run, resulting in a turn-around time of several hours to several days after the samples are collected.

Moreover, the above method is designed to perform certain operations under the guidance of a skilled technician who adds reagents, supervises processing, and moves samples step by step. Therefore, the laboratory test and method lead to high accuracy results but are time consuming and costly.

The foregoing is intended merely to aid in the understanding of the background of the present disclosure, and is not intended to mean that the present disclosure falls within the purview of the related art that is already known to those skilled in the art.

### SUMMARY OF THE INVENTION

Accordingly, the present disclosure has been made keeping in mind the above problems occurring in the related art, and an objective of the present disclosure is to provide an all-in-one kit for on-site molecular diagnosis, the kit enabling an ordinary person to perform molecular diagnosis without requiring the need for specialized equipment such as a centrifuge or pipette, ensuring low contamination operation, and being cost effective, and to provide a molecular diagnosis method using the same kit.

In order to achieve the above objective, according to one aspect of the present disclosure, there is provided an all-in-one kit for on-site molecular diagnosis, the kit including: a first component including a sample pre-processing part, a nucleic acid adsorption part, and a nucleic acid amplification part; and a second component including a detection part. The sample pre-processing part may include a transfer member configured to transfer a lysis buffer where a nucleic acid is dissolved and is configured to pre-process a sample. The nucleic acid adsorption part may be positioned on the sample pre-processing part and may be configured to be slidable onto the nucleic acid amplification part. The nucleic acid amplification part may be connected to the sample pre-processing part and may be configured to elute the nucleic acid from the nucleic acid adsorption part and to amplify the eluted nucleic acid. The detection part may be configured to transfer the nucleic acid amplified by the nucleic acid amplification part and to detect the nucleic acid.

Furthermore, the sample pre-processing part may further include a lysis buffer chamber positioned abutting the transfer member. The lysis buffer chamber may include the lysis buffer for dissolving the nucleic acid through lysis of the sample.

Furthermore, the lysis buffer chamber may be configured such that upper and lower ends thereof are destroyed by insertion of a sample collection tool.

Furthermore, the nucleic acid adsorption part may include a nucleic acid adsorption pad configured to bind to the nucleic acid present in the lysis buffer transferred from the transfer member.

Furthermore, the nucleic acid adsorption pad may be a silica membrane.

Furthermore, the transfer member may include a sample pad and a moving pad.

Furthermore, the sample pre-processing part may further include an absorption member. The absorption member may be configured to absorb the lysis buffer from the nucleic acid adsorption part.

Furthermore, the nucleic acid amplification part may include a reaction member containing an amplification reagent.

Furthermore, as the reaction member, a single or a plurality of reaction members may be provided.

Furthermore, when the plurality of reaction members may be provided, each of the plurality of reaction members may contain a different type of primer.

Furthermore, the detection part may include a single or a plurality of detection members configured to detect the nucleic acid.

Furthermore, the kit may diagnose at least one selected from the group consisting of corona virus, influenza virus, human immunodeficiency virus (HIV), variola virus, foot-and-mouth disease virus (FMDV), Ebola virus, dengue virus, Zika virus, human papillomavirus (HPV), bacterial pneumonia, tuberculosis, and gonorrhea.

Furthermore, the kit may further include a hot pack configured to control a temperature of the nucleic acid amplification part.

Furthermore, the kit may further include: a third component including a single or a plurality of needleless connector lower portions; and a fourth component including a single or a plurality of needleless connector upper portions. An elution buffer configured to elute the nucleic acid from the nucleic acid adsorption part or a running buffer configured to transfer the nucleic acid amplified from the nucleic acid amplification part to the detection part may be injected into the nucleic acid adsorption part through the needleless connector formed by combining the needleless connector lower portions and the needleless connector upper portions.

Furthermore, the kit may further include the sample collection tool configured to collect the sample.

According to another aspect of the present disclosure, there is provided a molecular diagnosis method using the kit, the molecular diagnosis method including: (a) generating the lysis buffer where the nucleic acid is dissolved by physically destroying an upper end of the lysis buffer chamber with the sample collection tool that has collected the sample containing the nucleic acid and by immersing the sample in the lysis buffer; (b) moving the lysis buffer where the nucleic acid is dissolved to the transfer member of the sample pre-processing part by physically destroying a lower end of the lysis buffer chamber with the sample collection tool, and binding the nucleic acid transferred along the transfer member to the nucleic acid adsorption part; (c) sliding the nucleic acid adsorption part to which the nucleic acid binds to a position on the nucleic acid amplification part; (d) eluting the nucleic acid from the nucleic acid adsorption part by injecting the elution buffer into the nucleic acid adsorption part, and transferring the eluted nucleic acid to the nucleic acid amplification part; (e) amplifying the nucleic acid located in the nucleic acid amplification part; and (f) detecting the nucleic acid by transferring the amplified nucleic acid to the detection part by injecting the running buffer into the nucleic acid amplification part.

Furthermore, at least one selected from the group consisting of the elution buffer of step (d) and the running buffer of step (f) may be injected with the needleless connector. The needleless connector is formed by combining the third component and the fourth component of the kit.

Furthermore, in the amplifying of the nucleic acid in step (e), the temperature of the nucleic acid amplification part is controlled by the hot pack.

Furthermore, the nucleic acid adsorption part includes a nucleic acid adsorption pad and the nucleic acid adsorption pad may be a silica membrane.

Furthermore, the molecular diagnosis method diagnoses at least one selected from the group consisting of corona virus, influenza virus, human immunodeficiency virus (HIV), variola virus, foot-and-mouth disease virus (FMDV), Ebola virus, dengue virus, Zika virus, human papillomavirus (HPV), bacterial pneumonia, tuberculosis, and gonorrhea.

The all-in-one kit for on-site molecular diagnosis and the molecular diagnosis method using the same according to the present disclosure are advantageous in that the kit enables an ordinary person to perform molecular diagnosis without requiring the need for specialized equipment such as a centrifuge or pipette, ensures low contamination operation, and is cost effective.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a schematic view illustrating an all-in-one kit for on-site molecular diagnosis according to the present disclosure;
FIG. 2 is an actual image of the all-in-one kit for on-site molecular diagnosis according to the present disclosure;
FIG. 3 is a schematic view illustrating the structure of an assembly of the all-in-one kit for on-site molecular diagnosis according to the present disclosure;
FIG. 4 is a schematic view illustrating the structures of a transfer member, an absorption member, and a nucleic acid adsorption part of the all-in-one kit for on-site molecular diagnosis according to the present disclosure;
FIG. 5 is a schematic view illustrating the structure of an assembly of the all-in-one kit for on-site molecular diagnosis according to the present disclosure;
FIG. 6 is a schematic view illustrating the structure of a first component into which a sample collection tool of the all-in-one kit for on-site molecular diagnosis according to the present disclosure is inserted;
FIG. 7 is a schematic view illustrating the structure of a second component of the all-in-one kit for on-site molecular diagnosis according to the present disclosure, including a detection part and a detection member;
FIG. 8 is a schematic view sequentially illustrating the steps of a molecular diagnosis method using an all-in-one kit for on-site molecular diagnosis according to the present disclosure;
FIG. 9 is a schematic view sequentially illustrating the steps of a molecular diagnosis method using the all-in-one kit for on-site molecular diagnosis according to the present disclosure; and
FIG. 10 illustrates the results of detecting SARS-CoV-2 virus by using an all-in-one kit for on-site molecular diagnosis prepared according to Example 1 and performing molecular diagnosis according to Example 3.

### DETAILED DESCRIPTION OF THE INVENTION

The present disclosure may be modified in various ways and implemented by various embodiments, so that specific embodiments will be described in detail. However, it is to be understood that the present disclosure is not limited to the specific exemplary embodiments, but includes all modifications, equivalents, and substitutions included in the spirit and the scope of the present disclosure. In the following description, detailed descriptions of known functions and components incorporated herein will be omitted when it may make the subject matter of the present disclosure unclear.

It will be further understood that, although the terms "first", "second", etc. may be used herein to describe various elements, these elements should not be limited by these terms. These terms are only used to distinguish one element from another. For example, a first element discussed below could be termed a second element without departing from the teachings of the present disclosure. Similarly, the second element could also be termed the first element.

Further, it will be understood that when an element is referred to as being "formed", "positioned", or "layered" on another element, it can be formed, positioned, or layered so as to be directly attached to the entire surface or one surface of the other element, or intervening elements may be present therebetween.

As used herein, the singular forms are intended to include the plural forms as well, unless the context clearly indicates otherwise. It will be further understood that the terms "comprise", "include", "have", etc. when used in this specification, specify the presence of stated features, integers, steps, operations, elements, components, and/or combinations of them but do not preclude the presence or addition of one or more other features, integers, steps, operations, elements, components, and/or combinations thereof.

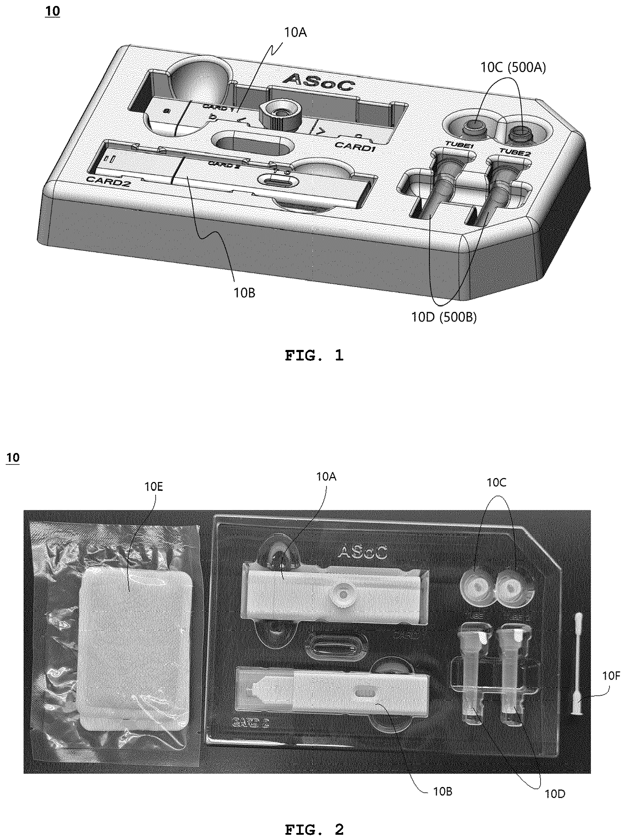

FIG. 1 is a schematic view illustrating an all-in-one kit 10 for on-site molecular diagnosis according to the present disclosure, and FIG. 2 is an actual image of the all-in-one kit 10 for on-site molecular diagnosis according to the present disclosure.

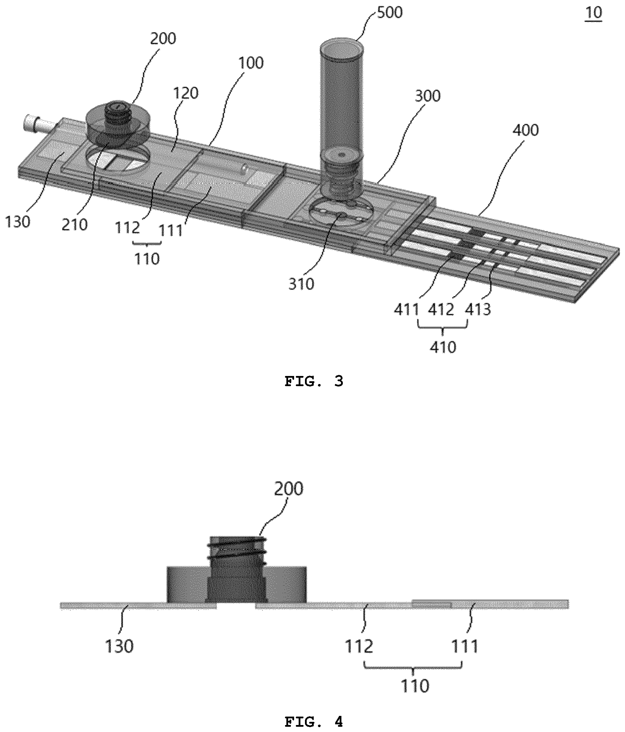

FIG. 3 is a schematic view illustrating the structure of an assembly of the all-in-one kit 10 for on-site molecular diagnosis according to the present disclosure, and FIG. 4 is a schematic view illustrating the structures of a transfer member 110, an absorption member 130, and a nucleic acid adsorption part 200 of the all-in-one kit 10 for on-site molecular diagnosis according to the present disclosure.

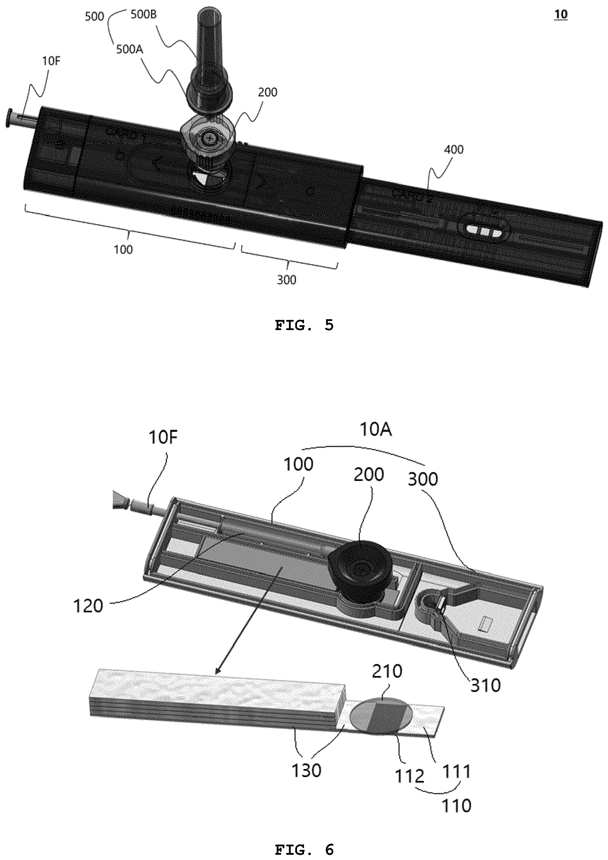

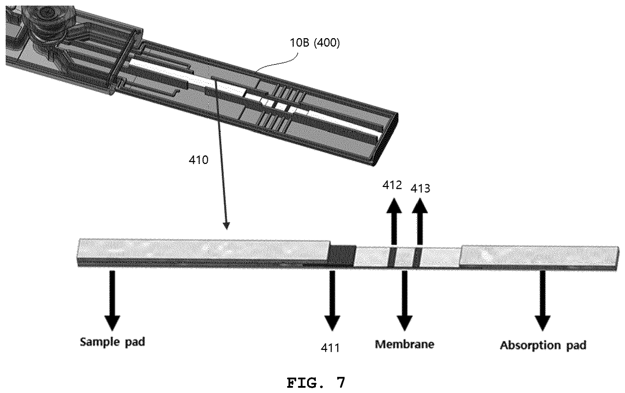

FIG. 5 is a schematic view illustrating the structure of an assembly of the all-in-one kit 10 for on-site molecular diagnosis according to the present disclosure, FIG. 6 is a schematic view illustrating the structure of a first component 10A into which a sample collection tool 10F of the all-in-one kit 10 for on-site molecular diagnosis according to the present disclosure is inserted, and FIG. 7 is a schematic view illustrating the structure of a second component 10B of the all-in-one kit 10 for on-site molecular diagnosis according to the present disclosure, including a detection part 400 and a detection member 410.

Hereinafter, the all-in-one kit 10 for on-site molecular diagnosis according to the present disclosure will be described with reference to FIGS. 1 to 7.

The present disclosure provides the all-in-one kit 10 for on-site molecular diagnosis, including: the first component 10A including a sample pre-processing part 100, the nucleic acid adsorption part 200, and a nucleic acid amplification part 300; and the second component 10B including the detection part 400.

### Sample pre-processing part 100

The sample pre-processing part 100 may include the transfer member 110 for transferring a lysis buffer where a nucleic acid is dissolved, and may pre-process a sample.

The sample pre-processing part 100 may further include a lysis buffer chamber 120 positioned abutting the transfer member 110.

The lysis buffer chamber 120 may include the lysis buffer for dissolving the nucleic acid through lysis of the sample.

The lysis buffer chamber 120 may be configured such that upper and lower ends thereof are destroyed by insertion of the sample collection tool 10F.

The transfer member 110 may include a sample pad 111 and a moving pad 112.

The sample pre-processing part 100 may further include the absorption member 130. The absorption member 130 may absorb the lysis buffer from the nucleic acid adsorption part 200.

### Nucleic acid adsorption part 200

The nucleic acid adsorption part 200 may be positioned on the sample pre-processing part 100, and may be slidable onto the nucleic acid amplification part 300.

The nucleic acid adsorption part 200 may include a nucleic acid adsorption pad 210 that binds to the nucleic acid present in the lysis buffer transferred from the transfer member 110.

The nucleic acid adsorption pad 210 may be a silica membrane.

### Nucleic acid amplification part 300

The nucleic acid amplification part 300 may be connected to the sample pre-processing part 100, and may elute the nucleic acid from the nucleic acid adsorption part 200 and amplify the eluted nucleic acid.

The nucleic acid amplification part 300 may include a reaction member 310 containing an amplification reagent.

As the reaction member 310, a single or a plurality of reaction members 310 may be provided.

When the plurality of reaction members 310 is provided, each of the plurality of reaction members 310 may contain a different type of primer.

### Detection part 400

The detection part 400 may transfer the nucleic acid amplified by the nucleic acid amplification part 300 and detect the nucleic acid.

The detection part 400 may include a single or a plurality of detection members 410 for detecting the nucleic acid.

### Needleless Connector 500

The all-in-one kit 10 for on-site molecular diagnosis according to the present disclosure may further include: a third component 10C including a single or a plurality of needleless connector lower portions 500A; and a fourth component 10D including a single or a plurality of needleless connector upper portions 500B.

An elution buffer for eluting the nucleic acid from the nucleic acid adsorption part 200 or a running buffer for transferring the nucleic acid amplified by the nucleic acid amplification part 300 to the detection part 400 may be injected into the nucleic acid adsorption part 200 through the needleless connector 500 formed by combining the needleless connector lower portions 500A and the needleless connector upper portions 500B.

The all-in-one kit 10 for on-site molecular diagnosis according to the present disclosure may diagnose at least one selected from the group consisting of corona virus, influenza virus, human immunodeficiency virus (HIV), variola virus, foot-and-mouth disease virus (FMDV), Ebola virus, dengue virus, Zika virus, human papillomavirus (HPV), bacterial pneumonia, tuberculosis, and gonorrhea.

The all-in-one kit 10 for on-site molecular diagnosis according to the present disclosure may further include a hot pack 10E for controlling the temperature of the nucleic acid amplification part 300.

The molecular diagnostic kit may further include the sample collection tool 10F for collecting the sample.

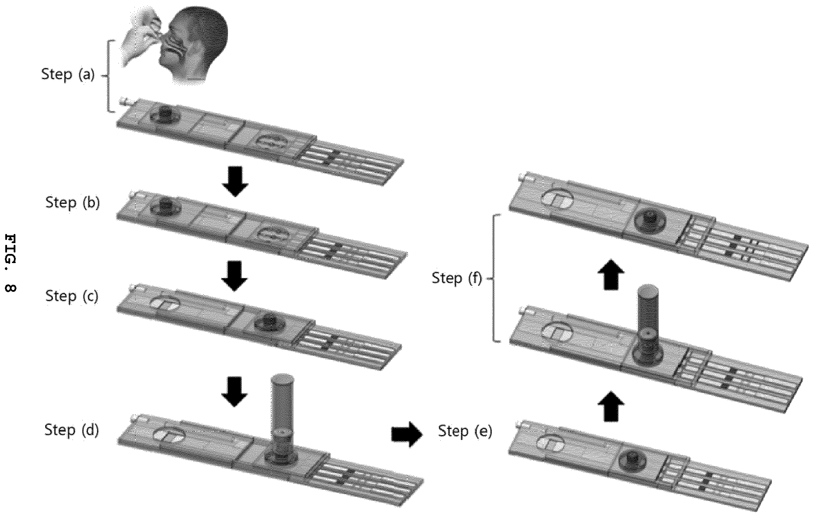

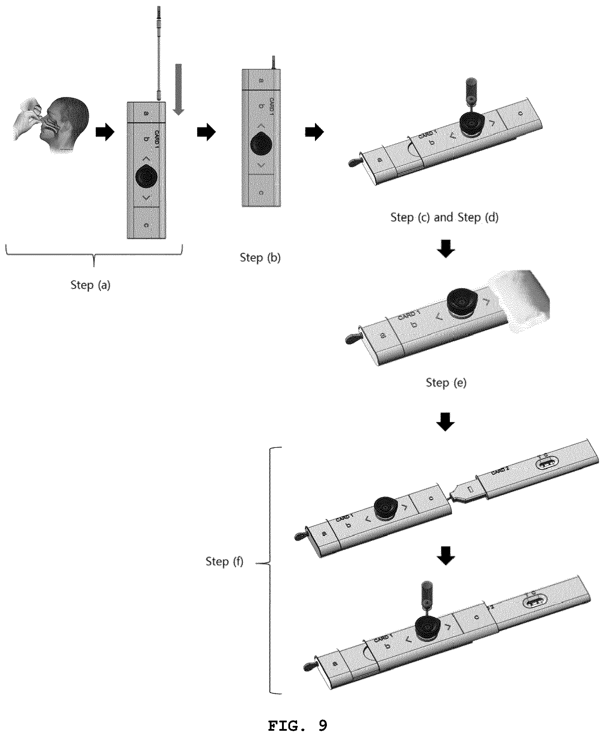

FIGS. 8 and 9 are schematic views each sequentially illustrating the steps of a molecular diagnosis method using an all-in-one kit for on-site molecular diagnosis according to the present disclosure.

Hereinafter, the molecular diagnosis method using the all-in-one kit for on-site molecular diagnosis according to the present disclosure will be described with reference to FIGS. 8 and 9.

First, a lysis buffer where a nucleic acid is dissolved is generated by physically destroying an upper end of a lysis buffer chamber 120 with a sample collection tool 10F that has collected a sample containing the nucleic acid and then immersing the sample in the lysis buffer (step (a)).

Then, the lysis buffer where the nucleic acid is dissolved is moved to a transfer member 110 of a sample pre-processing part 100 by physically destroying a lower end of the lysis buffer chamber 120 with the sample collection tool 10F, and the nucleic acid transferred along the transfer member 110 binds to a nucleic acid adsorption part 200 (step (b)).

The nucleic acid adsorption part 200 includes a nucleic acid adsorption pad 210. The nucleic acid adsorption pad 210 may be a silica membrane.

Then, the nucleic acid adsorption part 200 to which the nucleic acid binds is slid to a position on a nucleic acid amplification part 300 (step (c)).

Then, the nucleic acid is eluted from the nucleic acid adsorption part 200 by injecting an elution buffer into the nucleic acid adsorption part 200, and the eluted nucleic acid is transferred to the nucleic acid amplification part 300 (step (d)).

Then, the nucleic acid located in the nucleic acid amplification part 300 is amplified (step (e)).

In the amplifying of the nucleic acid in step (e), the temperature of the nucleic acid amplification part 300 may be controlled by a hot pack.

Finally, the nucleic acid is detected by transferring the amplified nucleic acid to a detection part 400 by injecting a running buffer into the nucleic acid amplification part 300 (step (f)).

At least one selected from the group consisting of the elution buffer of step (d) and the running buffer of step (f) is injected with a needleless connector 500. The needleless connector 500 may be formed by combining a third component 10C and a fourth component 10D of the all-in-one kit for on-site molecular diagnosis.

The molecular diagnosis method may diagnose at least one selected from the group consisting of corona virus, influenza virus, human immunodeficiency virus (HIV), variola virus, foot-and-mouth disease virus (FMDV), Ebola virus, dengue virus, Zika virus, human papillomavirus (HPV), bacterial pneumonia, tuberculosis, and gonorrhea.

### [Example]

Hereinafter, examples of the present disclosure will be described in detail. However, these examples are for illustrative purposes, and the scope of the present disclosure is not limited thereby.

### Example 1: All-in-one kit for on-site molecular diagnosis

Referring to FIGS. 1 and 2, an all-in-one kit 10 for on-site molecular diagnosis was manufactured, the kit including: a first component 10A including a sample pre-processing part 100, a nucleic acid adsorption part 200, and a nucleic acid amplification part 300; a second component 10B including a detection part 400; a third component 10C including two needleless connector lower portions 500A; a fourth component 10D including two needleless connector upper portions 500B; a hot pack 10E; and a sample collection tool 10F.

Referring to FIGS. 4 and 6, the sample pre-processing part 100 includes a transfer member 110 for transferring a lysis buffer where a nucleic acid is dissolved, and pre-processes a sample. The nucleic acid adsorption part 200 is positioned on the sample pre-processing part 100 and is slidable onto the nucleic acid amplification part 300. The nucleic acid amplification part 300 is connected to the sample pre-processing part 100, and elutes the nucleic acid from the nucleic acid adsorption part 200 and amplifies the eluted nucleic acid.

In this case, the sample pre-processing part 100 included a lysis buffer chamber 120 positioned abutting the transfer member 110, and an absorption member 130.

Referring to FIG. 7, the detection part 400 transfers the nucleic acid amplified by the nucleic acid amplification part 300 and detects the nucleic acid.

### Example 2: Molecular diagnosis after formation of assembly of all-in-one kit for on-site molecular diagnosis

Referring to FIG. 8, molecular diagnosis was performed using the all-in-one kit for on-site molecular diagnosis manufactured according to Example 1. A molecular diagnosis method is as follows.

First, an assembly as illustrated in FIG. 3 was formed by combining the first component 10A and the second component 10B of all-in-one kit 10 for on-site molecular diagnosis. At this point, the needleless connector 500 was formed by combining the third component 10C and the fourth component 10D.

Then, a lysis buffer where a nucleic acid is dissolved was generated by physically destroying an upper end of the lysis buffer chamber 120 with the sample collection tool 10F that has collected a sample containing the nucleic acid and then immersing the sample in the lysis buffer.

Then, the lysis buffer where the nucleic acid was dissolved was moved to a transfer member 110 of a sample pre-processing part 100 by physically destroying a lower end of the lysis buffer chamber 120 with the sample collection tool 10F, and the nucleic acid transferred along the transfer member 110 bound to the nucleic acid adsorption part 200.

Then, the nucleic acid adsorption part 200 to which the nucleic acid bound was slid to a position on the nucleic acid amplification part 300.

Then, the nucleic acid was eluted from the nucleic acid adsorption part 200 by injecting an elution buffer into the nucleic acid adsorption part 200, and the eluted nucleic acid was transferred to the nucleic acid amplification part 300. At this point, the elution buffer was injected with the needleless connector 500.

Then, the nucleic acid located in the nucleic acid amplification part 300 was amplified.

Finally, the nucleic acid was detected by transferring the amplified nucleic acid to the detection part 400 by injecting a running buffer into the nucleic acid amplification part 300 with the needless connector 500.

### Example 3: Formation of assembly of all-in-one kit for on-site molecular diagnosis during molecular diagnosis

Referring to FIG. 9, molecular diagnosis was performed using the all-in-one kit for on-site molecular diagnosis manufactured according to Example 1. A molecular diagnosis method is as follows.

First, a lysis buffer where a nucleic acid was dissolved was generated by physically destroying an upper end of the lysis buffer chamber 120 of the first component 10A with the sample collection tool 10F that has collected a sample containing the nucleic acid and then immersing the sample in the lysis buffer.

Then, the lysis buffer where the nucleic acid was dissolved was moved to a transfer member 110 of a sample pre-processing part 100 by physically destroying a lower end of the lysis buffer chamber 120 with the sample collection tool 10F, and the nucleic acid transferred along the transfer member 110 bound to the nucleic acid adsorption part 200. The step of binding the nucleic acid to the nucleic acid adsorption part 200 was performed by allowing the first component 10A to stand for 10 minutes.

Then, the needleless connector 500 was formed by combining the third component 10C and the fourth component 10D.

Then, the nucleic acid adsorption part 200 to which the nucleic acid bound was slid to a position on the nucleic acid amplification part 300.

Then, the nucleic acid was eluted from the nucleic acid adsorption part 200 by injecting an elution buffer into the nucleic acid adsorption part 200 with the needleless connector 500, and the eluted nucleic acid was transferred to the nucleic acid amplification part 300.

Then, the nucleic acid located in the nucleic acid amplification part 300 was amplified by covering the nucleic acid amplification part 300 of the first component 10A with the hot pack 10E and allowing the same to be left for 40 minutes.

Then, the hot pack 10E was removed, after which the first component 10A and the second component 10B were combined to form an assembly as illustrated in FIG. 5.

Finally, the nucleic acid was detected by transferring the amplified nucleic acid to the detection part 400 by injecting a running buffer into the nucleic acid amplification part 300 with the needless connector 500.

### [Test Example]

### Test Example 1: Evaluation of kit performance

Molecular diagnosis according to Example 3 was performed using the all-in-one kit for on-site molecular diagnosis manufactured according to Example 1.

In detail, in order to evaluate the performance of the all-in-one kit for on-site molecular diagnosis according to the present disclosure, SARS-CoV-2 heat inactivated virus acquired from BEI RESOURCE was used as a standard material. Two samples with viral loads of 1,000 copies/swab and 0 copies/swab, respectively, were diluted with Natural Nasal Swab Matrix (NNSM) and then each diluted solution was dropped onto a swab. After that, each swap sample was tested according to the procedure of using the all-in-one kit for on-site molecular diagnosis. The results were visually examined.

The amplified nucleic acid was transferred to the detection part 400 by injecting a running buffer into the nucleic acid amplification part 300 with the needless connector 500. After 5 minutes, the results were examined.

The examination of the results was performed by visually observing a color line of the detection part 400. When two lines of a test line T and a control line C were colored, the sample was determined as positive, and when one line of the control line C is colored, the sample was determined as negative.

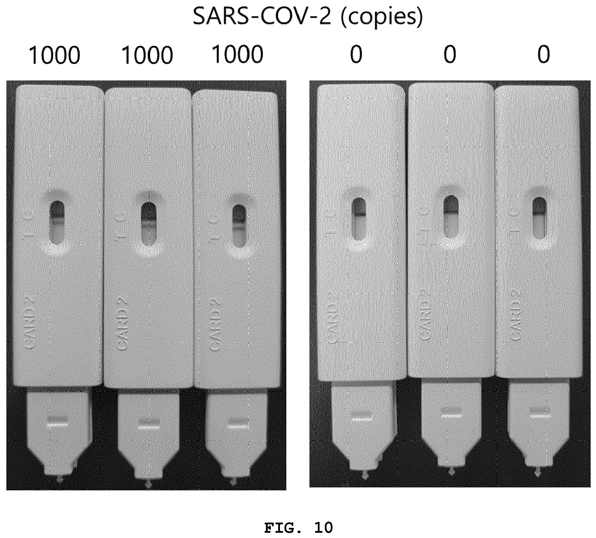

FIG. 10 illustrates the results of detecting SARS-CoV-2 virus by using the all-in-one kit for on-site molecular diagnosis prepared according to Example 1 and performing molecular diagnosis according to Example 3.

Referring to FIG. 10, the results reveal that the all-in-one kit for on-site molecular diagnosis according to the present disclosure can detect up to 1,000 copies of SARS-CoV-2 nucleic acids in the samples.

The aforementioned descriptions are only for illustrative purposes, and it will be apparent that those of skill in the art can make various modifications thereto without changing the technical spirit and essential features of the present disclosure. Thus, it should be understood that the embodiments described above are merely for illustrative purposes and not for limitation purposes in all aspects. For example, each component described as a single type can be implemented in a distributed type, and components described as distributed can be implemented in a combined form. The scope of the present disclosure is defined by the accompanying claims rather than the description which is presented above. Moreover, the present disclosure is intended to cover not only the embodiments, but also various alternatives, modifications, equivalents, and other embodiments that may be included within the spirit and scope of the present disclosure as defined by the accompanying claims.

## Claims

1. An all-in-one kit for on-site molecular diagnosis, the kit comprising:
a first component comprising a sample pre-processing part, a nucleic acid adsorption part, and a nucleic acid amplification part; and
a second component comprising a detection part,
wherein the sample pre-processing part comprises a transfer member configured to transfer a lysis buffer where a nucleic acid is dissolved and is configured to pre-process a sample,
the nucleic acid adsorption part is positioned on the sample pre-processing part and is configured to be slidable onto the nucleic acid amplification part,
the nucleic acid amplification part is connected to the sample pre-processing part and is configured to elute the nucleic acid from the nucleic acid adsorption part and to amplify the eluted nucleic acid, and
the detection part is configured to transfer the nucleic acid amplified by the nucleic acid amplification part and to detect the nucleic acid.

2. The kit of claim 1, wherein the sample pre-processing part further comprises a lysis buffer chamber positioned abutting the transfer member, wherein the lysis buffer chamber comprises the lysis buffer for dissolving the nucleic acid through lysis of the sample.

3. The kit of claim 2, wherein the lysis buffer chamber is configured such that upper and lower ends thereof are destroyed by insertion of a sample collection tool.

4. The kit of claim 1, wherein the nucleic acid adsorption part comprises a nucleic acid adsorption pad configured to bind to the nucleic acid present in the lysis buffer transferred from the transfer member.

5. The kit of claim 4, wherein the nucleic acid adsorption pad is a silica membrane.

6. The kit of claim 1, wherein the transfer member comprises a sample pad and a moving pad.

7. The kit of claim 1, wherein the sample pre-processing part further comprises an absorption member, wherein the absorption member is configured to absorb the lysis buffer from the nucleic acid adsorption part.

8. The kit of claim 1, wherein the nucleic acid amplification part comprises a reaction member containing an amplification reagent.

9. The kit of claim 8, wherein as the reaction member, a single or a plurality of reaction members is provided.

10. The kit of claim 9, wherein when the plurality of reaction members is provided, each of the plurality of reaction members contains a different type of primer.

11. The kit of claim 1, wherein the detection part comprises a single or a plurality of detection members configured to detect the nucleic acid.

12. The kit of claim 1, wherein the kit diagnoses at least one selected from the group consisting of corona virus, influenza virus, human immunodeficiency virus (HIV), variola virus, foot-and-mouth disease virus (FMDV), Ebola virus, dengue virus, Zika virus, human papillomavirus (HPV), bacterial pneumonia, tuberculosis, and gonorrhea.

13. The kit of claim 1, further comprising a hot pack configured to control a temperature of the nucleic acid amplification part.

14. The kit of claim 1, further comprising:
a third component comprising a single or a plurality of needleless connector lower portions; and
a fourth component comprising a single or a plurality of needleless connector upper portions,
wherein an elution buffer configured to elute the nucleic acid from the nucleic acid adsorption part or a running buffer configured to transfer the nucleic acid amplified from the nucleic acid amplification part to the detection part is injected into the nucleic acid adsorption part through a needleless connector formed by combining the needleless connector lower portions and the needleless connector upper portions.

15. The kit of claim 1, further comprising the sample collection tool configured to collect the sample.

16. A molecular diagnosis method using the kit of claim 1, the molecular diagnosis method comprising:
(a) generating the lysis buffer where the nucleic acid is dissolved by physically destroying an upper end of the lysis buffer chamber with the sample collection tool that has collected the sample containing the nucleic acid and by immersing the sample in the lysis buffer;
(b) moving the lysis buffer where the nucleic acid is dissolved to the transfer member of the sample pre-processing part by physically destroying a lower end of the lysis buffer chamber with the sample collection tool, and binding the nucleic acid transferred along the transfer member to the nucleic acid adsorption part;
(c) sliding the nucleic acid adsorption part to which the nucleic acid binds to a position on the nucleic acid amplification part;
(d) eluting the nucleic acid from the nucleic acid adsorption part by injecting the elution buffer into the nucleic acid adsorption part, and transferring the eluted nucleic acid to the nucleic acid amplification part;
(e) amplifying the nucleic acid located in the nucleic acid amplification part; and
(f) detecting the nucleic acid by transferring the amplified nucleic acid to the detection part by injecting the running buffer into the nucleic acid amplification part.

17. The molecular diagnosis method of claim 16, wherein at least one selected from the group consisting of the elution buffer of step (d) and the running buffer of step (f) is injected with the needleless connector, wherein
the needleless connector is formed by combining the third component and the fourth component of the kit.

18. The molecular diagnosis method of claim 16, wherein in the amplifying of the nucleic acid in step (e), the temperature of the nucleic acid amplification part is controlled by the hot pack.

19. The molecular diagnosis method of claim 16, wherein the molecular diagnosis method diagnoses at least one selected from the group consisting of corona virus, influenza virus, human immunodeficiency virus (HIV), variola virus, foot-and-mouth disease virus (FMDV), Ebola virus, dengue virus, Zika virus, human papillomavirus (HPV), bacterial pneumonia, tuberculosis, and gonorrhea.
